(19) Europäisches Patentamt

European Patent Office

Office européen des brevets

(11) **EP 1 509 496 B1**

(12) **EUROPÄISCHE PATENTSCHRIFT**

(45) Veröffentlichungstag und Bekanntmachung des Hinweises auf die Patenterteilung:
**27.12.2006 Patentblatt 2006/52**

(21) Anmeldenummer: **03755040.7**

(22) Anmeldetag: **19.05.2003**

(51) Int Cl.:
*C07C 263/10* (2006.01)    *B01J 19/24* (2006.01)

(86) Internationale Anmeldenummer:
**PCT/EP2003/005232**

(87) Internationale Veröffentlichungsnummer:
**WO 2003/099770 (04.12.2003 Gazette 2003/49)**

(54) **VERFAHREN ZUR HERSTELLUNG VON ISOCYANATEN UND REAKTIONSVORRICHTUNG**

METHOD FOR PRODUCING ISOCYANATES AND REACTION APPARATUS

PROCEDE DE FABRICATION D'ISOCYANATES ET APPAREILLAGE DE REACTION

(84) Benannte Vertragsstaaten:
**AT BE BG CH CY CZ DE DK EE ES FI FR GB GR HU IE IT LI LU MC NL PT RO SE SI SK TR**

(30) Priorität: **23.05.2002 DE 10222968**

(43) Veröffentlichungstag der Anmeldung:
**02.03.2005 Patentblatt 2005/09**

(73) Patentinhaber: **BASF Aktiengesellschaft**
**67056 Ludwigshafen (DE)**

(72) Erfinder:
• **WÖLFERT, Andreas**
**74906 Bad Rappenau (DE)**
• **MÜLLER, Christian**
**68167 Mannheim (DE)**
• **STRÖFER, Eckhard**
**68163 Mannheim (DE)**
• **BRODHAGEN, Andreas**
**67125 Dannstadt-Schauernheim (DE)**

(56) Entgegenhaltungen:
**EP-A- 0 065 727        EP-A- 0 322 647**
**WO-A-99/40059        FR-A- 1 482 314**
**GB-A- 1 120 770**

**Beschreibung**

Die Erfindung betrifft ein neues Verfahren zur Herstellung von Isocyanaten durch Umsetzung von primären Aminen mit Phosgen durch einstufige Umsetzung in einer Kaskade von Reaktoren mit zwischengeschalteter Gasphasenabtrennung.

Es sind in der Literatur bereits verschiedene Verfahren zur Herstellung von Isocyanaten durch Umsetzung von Aminen mit Phosgen beschrieben.

DE-A-958 558 und DE-A-958 086 beschreiben ein zweistufiges Verfahren, wobei die Reaktionspartner bei der Heißphosgenierung von unten her durch ein aufrecht oder schräg stehendes Rohr geleitet werden, wodurch Phosgen durch den Druck der im Rohr stehenden Flüssigkeitssäule stärker in Lösung gehalten und somit eine höher Phosgenkonzentration erzeugt wird, die sich beschleunigend auf den Reaktionsverlauf auswirkt. Nachteilig ist hier die schlechte Raum-Zeit-Ausbeute bei der Kalt-Heißphosgenierung.

DE-A-1 768 439 beschreibt eine einstufige Phosgenierung in einem Rohrreaktor in der Flüssigphase, dadurch gekennzeichnet, dass die eingestellten Temperaturen so hoch sind, dass das zugehörige Hydrochlorid nicht stabil in der Lösung vorliegt. Nachteilig ist, dass um das Phosgen bei den gewählten Temperaturen in der Lösung zu halten, so hohe Drücke erforderlich sind, dass die Druckausführung der Apparate sehr aufwendig ist. Durch die hohen Temperaturen ergeben sich auch eine hohe Bildung von unerwünschten Nebenkomponenten.

DE-A-2 112 181 beschreibt die Phosgenierung in einem mit Füllkörpern gefüllten Rohrreaktor im Gleichstrom, wobei die Strömung im Rohrreaktor im Übergangsregime zwischen laminarer und pulsierender Strömung, und wobei ein Teil der fertigen Reaktionslösung wieder in den Rohrreaktor zurückgeführt wird. Nachteilig ist die Verstopfungsneigung des Verfahrens durch Anlagerungen an den Füllkörpern.

DE-A-31 21 036 beschreibt ein kontinuierliches Verfahren zur Herstellung von Isocyanaten, gekennzeichnet durch einstufige Temperaturführung in einer Kombination von Mischdüse und Rohrreaktor. Nachteilig bei dem Verfahren ist, dass durch den Verzicht auf eine Zwischenentgasung das Volumen des Rohrreaktors sehr groß ist.

GB-A-1 120 770 offenbart die kontinuierliche Herstellung von Isocyanaten durch einstufige Umsetzung von primären Aminen mit Phosgen in einer Kaskade aus 3 Reaktoren, wobei nach jedem Reaktor die bei der Umsetzung entstehende Gasphase abgetrennt wird, und die Flüssigphase dem nächsten Reaktor zugeführt wird. Dabei ist das Volumen des ersten Reaktors geringer als das Gesamtvolumen pro Anzahl Reaktoren.

Es ist weiterhin bekannt, dass die Verwendung eines hohen Phosgenüberschusses gegenüber dem verwendeten Amin zu hohen Selektivitäten bezüglich des hergestellten Isocyanates führt und somit einen entscheidenden Einfluss auf die Wirtschaftlichkeit des Herstellungsverfahrens hat. Mit zunehmendem Verhältnis von Phosgen zu Aminogruppen steigt tendenziell der Phosgen-Hold-Up der Anlage und das Anlagenvolumen. Andererseits wird aufgrund der Giftigkeit von Phosgen ein möglichst geringer Phosgen-Hold-Up und ein möglichst kompakter Bau der Anlage angestrebt. Dies stellt gleichzeitig eine Reduzierung der Investitionskosten der Anlage und somit eine Verbesserung der Wirtschaftlichkeit des Verfahrens dar.

Aufgabe der Erfindung war es daher, ein Verfahren zur Herstellung von Isocyanaten bereitzustellen, welches es gestattet, dass die resultierenden Reaktionen unter hoher Selektivität und hoher Raum-Zeit-Ausbeute durchgeführt werden, so dass das Verfahren räumlich kompakt aufgebaut werden kann.

Überraschend wurde gefunden, dass sich das Gesamtreaktionsvolumen unter Beibehaltung der Ausbeute der Anlage gegenüber der bekannten einstufigen Durchführung des Verfahrens bzw. gegenüber der mehrstufigen Durchführung des Verfahrens als Kalt- Heißphosgenierung, reduzieren lässt, wenn die Reaktion hinsichtlich der Temperaturführung einstufig in einer Kaskade von Rohrreaktoren durchgeführt wird, wobei nach jedem Reaktor die bei der Umsetzung entstehende Gasphase abgetrennt wird, und nur die Flüssigphase dem nächsten Reaktor bzw. der Produktreinigung zugeführt wird, und wobei das Reaktionsvolumen des ersten Reaktors geringer ist als das Gesamtreaktionsvolumen pro Anzahl der Reaktoren.

Gegenstand der Erfindung ist daher ein Verfahren zur kontinuierlichen Herstellung von Isocyanaten durch einstufige Umsetzung von primären Aminen mit Phosgen in einer Kaskade aus n Reaktoren, wobei n eine natürliche Zahl von 2 bis 20 darstellt, dadurch gekennzeichnet, dass die Vermischung und Umsetzung der Edukte in einem Schritt in einen Temperatur-Bereich von 60 bis 200°C durchgeführt wird und nach jedem Reaktor die bei der Umsetzung entstehende Gasphase vom Umsetzungsgemisch abgetrennt und die verbleibende Flüssigphase dem nächsten Reaktor zugeführt wird, wobei das Volumen des ersten Reaktors folgender Ungleichung genügt:

$$\text{Volumen}_{\text{Reaktor 1}} < (\text{Volumen}_{\text{Summe der Reaktoren 1 bis n}}) / n.$$

Gegenstand der Erfindung ist ferner eine Reaktionsvorrichtung zur kontinuierlichen Herstellung von Isocyanaten durch einstufige Umsetzung von primären Aminen mit Phosgen, aufgebaut aus einer Kaskade aus n Reaktoren, bevorzugt Rohrreaktoren, wobei n eine natürliche Zahl von 2 bis 20 darstellt, dadurch gekennzeichnet, dass die Reaktoren eine Phasentrennvorrichtung, die zur Abtrennung der bei der Umsetzung entstehende Gasphase geeignet ist, aufweisen, wobei die Phasentrennvorrichtung in den Reaktor integriert ist oder getrennt als Behälter aufgeführt ist, und das Volumen des ersten Reaktors folgender Ungleichung genügt:

$$\text{Volumen }_{\text{Reaktor 1}} < (\text{Volumen }_{\text{Summe der Reaktoren 1 bis n}}) \ / \ n,$$

wobei zwischen Phasentrennvorrichtung und Reaktor eine Druckreduziereinrichtung verwendet wird.

Ebenfalls ist Gegenstand der Erfindung die Verwendung einer vorstehend beschriebenen Reaktionsvorrichtung zur Herstellung von aromatischen oder aliphatischen Isocyanaten durch Phosgenierung von primären Aminen.

In einer bevorzugten Ausführungsform ist n eine natürliche Zahl von 2 bis 5, besonders bevorzugt ist n 2 oder 3.

Es ist weiterhin bevorzugt, dass im erfindungsgemäßen Verfahren das Volumen des ersten Reaktors das 0,1- bis 0,9-fache, mehr bevorzugt 0,3- bis 0,85-fache, insbesondere 0,5 bis 0,8-fache, bezogen auf das Gesamtvolumen durch Anzahl der Reaktoren [(Volumen $_{\text{Summe der Reaktoren 1 bis n}}$) / n] beträgt.

Bei der bevorzugten Ausführungsform, in der n zwei bedeutet, ist es bevorzugt, dass das Volumen des ersten Reaktors 5 bis weniger als 50 % , mehr bevorzugt 5 bis 45 %, Noch mehr bevorzugt 7 bis weniger als 40 %, besonders bevorzugt 10 bis 30 % des Gesamtvolumens beträgt.

Bei den die Kaskade bildenden Reaktoren kann es sich um alle üblichen Reaktoren handeln, die zur kontinuierlichen Phosgenierung geeignet sind. Bevorzugt verwendet werden Rohrreaktoren.

In einer bevorzugten Ausführungsform handelt es sich bei den Reaktoren um senkrecht angeordnete Reaktionsrohre, wobei das Umsetzungsgemisch von unten nach oben die Reaktionsrohre durchläuft.

Der Rohrreaktor wird bevorzugt entweder über seine Mantelfläche oder über im Rohrreaktor enthaltene Heizelemente z.B. Heizschlangen, bzw. Heizrohre temperiert. Zur Einengung der Verweilzeitverteilung kann der Rohrreaktor durch Lochböden segmentiert werden. In einer weiteren bevorzugten Ausführungsform ist der Rohrreaktor durch ein Längen- (L) zu Durchmesserverhältnis (D) von L/D > 6, bevorzugt durch L/D > 10 definiert. Um Produktionsanlagen mit hoher Anlagenkapazität zu bauen, können auch mehrere Reaktorrohre parallel geschalten werden.

Das erfindungsgemäße Verfahren wird einstufig durchgeführt. Darunter ist zu verstehen, dass die Vermischung und Umsetzung der Edukte in einem Schritt in einem Temperaturbereich von 60 bis 200°C durchgeführt wird. Im Unterschied hierzu werden viele aus dem Stand der Technik bekannte Verfahren zweistufig durchgeführt, d.h. das Vermischen der Edukte erfolgt bei etwa 30°C (hier bildet sich Carbamylchlorid, diese Stufe wird oft als Kaltphosgenierung bezeichnet) und anschließend werden die gemischten Edukte bei etwa 120 bis 200°C erhitzt (hier wird das Carbamylchlorid zu Isocyanat gespalten, diese Stufe wird oft als Heißphosgenierung bezeichnet).

Im erfindungsgemäßen Verfahren erfolgt die Vermischung der Reaktanten in einer Mischeinrichtung, die sich durch eine hohe Scherung des durch die Mischeinrichtung geführten Reaktionsstromes auszeichnet. Bevorzugt werden als Mischeinrichtung eine Rotationsmischeinrichtung, eine Mischpumpe oder eine Mischdüse verwendet, die dem Reaktor vorangestellt ist. Besonders bevorzugt wird eine Mischdüse verwendet.

In einer bevorzugten Ausführungsform erfolgt die Umsetzung von Amin mit Phosgen bei Absolutdrücken von 0,9 bar bis 400 bar, bevorzugt von 3 bis 35bar. Das molare Verhältnis von Phosgen zu eingesetzten Aminogruppen beträgt im allgemeinen 1 : 1 bis 12 : 1, bevorzugt von 1,25 : 1 bis 5 : 1. Die Gesamtverweilzeit in den Reaktoren beträgt in allgemeinen 10 Sekunden bis 3 Stunden.

In einer bevorzugten Ausführungsform erfolgt die Abtrennung der Gasphase vom Umsetzungsgemisches am Ende eines Reaktors mittels einer Phasentrennvorrichtung. Als Phasentrennvorrichtung können ungerührte Behälter verwendet werden.

Die Phasentrennung kann durch eine in den Rohrreaktor eingebaute Phasentrennvorrichtung oder durch eine separat gebaute Phasentrennvorrichtung durchgeführt werden. Vorzugsweise wird die Phasentrennung separat in einem Behälter durchgeführt, der z.B. als stehender oder liegender Behälter ausgeführt ist, und in dem die Trennung der beiden Phasen durch eine Beruhigung der Strömung durch den Behälter erreicht wird.

Für die Durchführung der Phasentrennung ist es aber vorteilhaft, dass der Flüssigkeitsstand auch bei verschiedenen Lastbedingungen (z.B. Teillast) eingestellt werden kann. Der Flüssigkeitstand kann geregelt oder ungeregelt sein, oder auch durch einen Überlauf zur Abtrennung der flüssigen Phase sichergestellt werden. Vorzugsweise wird der Flüssigkeitsstand durch Regelung eingestellt. Alternativ kann ein Zentrifugalabscheider zur Phasenabtrennung eingesetzt werden.

Vorteilhaft ist auch eine temperierbare Ausführung des Phasentrennbehälters über die Behälterwand oder über Heiz/Kühleinrichtungen im Behälter oder über einen im Kreislauf mit dem Behälter geschalteten externen Wärmetauscher. Dadurch kann ein Auskühlen der Reaktionsmischung im Phasentrennbehälter sicher verhindert werden. Eine Abkühlung könnte zum Ausfallen von schwerlöslichen Feststoffen aus der Reaktionslösung und zum Verstopfen des Apparates führen.

Es hat sich weiterhin als vorteilhaft erwiesen, zwischen den Phasentrennvorrichtung und den Reaktoren, bevorzugt Rohrreaktoren, und/oder am Ende der letzten Phasentrennvorrichtung Druckreduziereinrichtungen, bevorzugt Druckreduzierventile, zu verwenden. Durch den Druckhaltungsmechanismus wird ein frühzeitiger Übergang des Phosgens aus der Reaktionslösung in die Gasphase verhindert. Es ist aber bekannt, dass ein möglichst hoher Überschuss an Phosgen zu guten Ausbeuten führt, was durch den Prozess des Übergehens des Phosgen in die Gasphase verhindert wird.

Ferner kann es zur Erreichung eines höheren Endumsatzes sinnvoll sein, dass der Austrag aus dem letzten Reaktor der Kaskade einer Nachreaktionsvorrichtung zur Nachreaktion zugeführt wird. Als Nachreaktionsvorrichtung ist im allgemeinen jede Reaktionsvorrichtung geeignet, worin eine Zersetzung von in der Kaskade noch nicht umgesetzten Carbamylchlorid und Aminhydrochlorid erfolgen kann. Beispiele für eine solche Vorrichtung sind eine Destillationskolonne und/oder eine Blasensäule. Bevorzugt wird eine Destillationskolonne, besonders bevorzugt einer Reaktivdestillationskolonne, verwendet.

In einer bevorzugten Ausführungsform wird der Austrag einer Destillationskolonne im Mittelteil zugeführt. In der Kolonne erfolgt eine Zersetzung des noch nicht umgesetzten Carbamylchlorids und Aminhydrochlorids. Die Flüssigphase, beispielsweise Phosgen, Isocyanat, Lösungsmittel und Schwersieder werden innerhalb der Kolonne von oben nach unten und die Gasphase, beispielsweise Chlorwasserstoff und Phosgen, von unten nach obengeleitet.

In der Nachreaktionsvorrichtung werden die Reaktionsbedingungen im allgemeinen so gewählt, dass eine Zersetzung des noch nicht umgesetzten Carbamylchlorids und Aminhydrochlorids erfolgt. Falls es sich um eine Destillationskolonne handelt, wird üblicherweise ein Druck eingestellt, der dem Druck in den vorangegangenen Reaktoren im wesentlichen gleicht, im allgemeinen liegt der Druck bei 1 bis 10 bar. In der Destillationskolonne herrscht im allgemeinen eine Temperatur zwischen 60 und 300°C.

Durch die Reduktion des Gesamtreaktionsvolumen lässt sich die Anlage wesentlich kompakter bauen. Dies stellt im Hinblick auf die Gefährlichkeit des in der Anlage gehandhabten Phosgens eine erhebliche Erhöhung der Sicherheit der Anlage und gleichzeitig eine Reduzierung der Investitionskosten dar.

Für das erfindungsgemäße Verfahren kann ein beliebiges primäres Amin oder ein Gemisch aus zwei oder mehr dieser Amine eingesetzt werden. Bevorzugt werden aromatische Amine, insbesondere solche der Diaminodiphenylmethanreihe oder deren höheren Homologen eingesetzt. Beispiele sind Methylendiphenylamin (einzelne Isomere, Isomerengemisch und/oder Oligomere davon), Toluylendiamin, n-Pentylamin, 6-Methyl-2-heptanamin, Cyclopentylamin, R,S-1-Phenylethylamin, 1-Methyl-3-phenylpropylamin, 2,6-Xylidin, 2-Dimethylaminoethylamin, 2-Diisopropylaminoethylamin, C11-Neodiamin, Isophorondiamin, 1,6-Hexamethylendiamin, Naphtyldiamin, 3,3'-Diaminodiphenylsulfon und 4-Aminomethyl-1,8-octan-diamin.

Das erfindungsgemäße Verfahren eignet sich im allgemeinen zur Herstellung von beliebigen Isocyanaten. Besonders vorteilhaft kann das Verfahren zur Herstellung von Methylen(diphenylisocyanat) (MDI) und Toluylendiisocyanat (TDI) angewandt werden.

Dem erfindungsgemäßen Verfahren kann ein zusätzliches inertes Lösungsmittel beigesetzt werden. Dieses zusätzliche inerte Lösungsmittel ist üblicherweise ein organisches Lösungsmittel oder Gemische davon. Dabei sind Chlorbenzol, Dichlorbenzol, Trichlorbenzol, Toluol, Hexan, Diethylisophthalat (DEIP), Tetrahydrofuran (THF), Dimethylformamid (DMF), Benzol und deren Gemische bevorzugt. Auch kann das Isocyanat, das in der Anlage hergestellt wird als Lösungsmittel verwendet werden. Besonders bevorzugt ist Chlorbenzol.

Nach der Reaktion wird das Stoffgemisch bevorzugt mittels Rektifikation in Isocyanat(e), Lösungsmittel, Phosgen und Chlorwasserstoff aufgetrennt. Geringe Mengen von Nebenprodukten, die im Isocyanat(e) verbleiben, können mittels zusätzlicher Rektifikation oder auch Kristallisation vom erwünschten Isocyanat(e) getrennt werden.

Je nach Wahl der Reaktionsbedingungen kann das Produkt inertes Lösungsmittel, Carbamoylchlorid und/oder Phosgen enthalten und nach den bekannten Methoden weiterverarbeitet werden (siehe z.B. WO 99/40059).

Die für das erfindungsgemäße Verfahren beschriebenen bevorzugten Ausführungsformen gelten entsprechend auch für die erfindungsgemäße Reaktionsvorrichtung.

Die Erfindung soll durch nachstehende Beispiele veranschaulicht werden.

Beispiel 1

In einer koaxialen Doppelrohrmischdüse wurden über das innere Rohr ein TDA-Lösungsstrom bei einer Geschwindigkeit von 35 m/s, der aus 28.04 kg/h Monochlorbenzol und 7.01 kg/h TDA besteht, wobei das TDA zu 80 Gewichts-% aus 2,4-TDA und zu 20 Gewichts-% aus 2,6-TDA besteht, mit 56.75 kg/h flüssigem Phosgen vermischt, das über den äußeren Ringspalt mit einer Geschwindigkeit von 15 m/s zugeführt wird. Die Temperaturen der Eduktströme wurden so eingestellt, dass der aus der Mischdüse austretende Austrittsstrom eine Temperatur von 100 °C besaß. Dieser Strom wurde dann durch einen senkrechtstehendes Rohr mit einem Volumen von 2 l (Liter) und einem Längenzu Durchmesserverhältnis von 8 geleitet. Durch eine elektrische Begleitbeheizung wurde die Rohrwand auf eine Temperatur von 140 °C eingestellt. Am Kopf des Rohrreaktors wurde die Flüssigphase von der HCl und Phosgen enthaltenden Gasphase abgetrennt. Die Flüssigphase wurde durch einen weiteren Rohrreaktor mit einem Volumen von 10 l und einem Längen- zu Durchmesserverhältnis von 8 geleitet. Durch eine elektrische Begleitbeheizung wurde auch hier die Rohrwand auf eine Temperatur von 140 °C eingestellt. Am Kopf des Rohrreaktors wurde wieder die Flüssigphase von der HCl und Phosgen enthaltenden Gasphase abgetrennt. Nach der destillativen Entfernung des Phosgens und des Chlorbenzols aus der Flüssigphase wurde TDI bei einer Reinheit von ca. 99,1 % (GC) und einer Ausbeute von 97% in Bezug auf den eingesetzten TDA-Strom gewonnen. Vor und nach der Synthese musste die Apparatur kontinuierlich mit MCB vorgespült werden. Das Reaktionsvolumen betrug insgesamt ca. 12 1. Der Druck in den Rohrreaktoren wurde auf 4.5 bar absolut eingestellt.

Beispiel 2 (Vergleichsbeispiel)

In der koaxialen Doppelrohrmischdüse der Versuchsanlage wurden über das innere Rohr ein TDA-Lösungsstrom bei einer Geschwindigkeit von 35 m/s, der aus 28.04 kg/h Monochlorbenzol und 7.01 kg/h TDA besteht, wobei das TDA zu 80 Gewichts-% aus 2,4-TDA und zu 20 Gewichts-% aus 2,6-TDA besteht, mit 56.75 kg/h flüssigem Phosgen vermischt, das über den äußeren Ringspalt mit einer Geschwindigkeit von 15 m/s zugeführt wurde. Die Temperaturen der Eduktströme wurden so eingestellt, dass der aus der Mischdüse austretende Austrittsstrom eine Temperatur von 100°C besitzt. Dieser Strom wurde dann durch einen senkrechtstehendes Rohr mit einem Volumen von 10 l (Liter) und einem Längen- zu Durchmesserverhältnis von 8 geleitet. Durch eine elektrische Begleitbeheizung wurde die Rohrwand auf eine Temperatur von 140°C eingestellt. Am Kopf des Rohrreaktors wird die Flüssigphase von der HCl und Phosgen enthaltenden Gasphase abgetrennt. Die Flüssigphase wurde einem weiteren Rohrreaktor gleicher Bauweise mit ebenfalls 10 1 Volumen, einem Längen- zu Durchmesserverhältnis von 8 und einer Rohrwandtemperatur von 140°C zugeführt. Am Kopf des Rohrreaktors wurde wieder die Flüssigphase von der HCl und Phosgen enthaltenden Gasphase abgetrennt. Nach der destillativen Entfernung des Phosgens und des Chlorbenzols aus der Flüssigphase wurde TDI bei einer Reinheit von ca. 99,1 % (GC) und einer Ausbeute von 97 % in Bezug auf den eingesetzten TDA-Strom gewonnen. Der Druck in den Rohrreaktoren wurde auf 4.5 bar absolut eingestellt. Vor und nach der Synthese musste die Apparatur kontinuierlich mit MCB vorgespült werden. Das Reaktionsvolumen betrug insgesamt ca. 20 1.

Ergebnis: In Vergleichsbeispiel 2 ergab sich gegenüber dem Beispiel 1 ein um 66.6 % größeres Reaktionsvolumen bei gleicher Ausbeute des Verfahrens.

Beispiel 3

Wie in Beispiel 1 wurden in einer koaxialen Doppelrohrmischdüse über das innere Rohr ein TDA-Lösungsstrom bei einer Geschwindigkeit von 35 m/s, der aus 28.04 kg/h Monochlorbenzol und 7,01 kg/h TDA besteht, wobei das TDA zu 80 Gewichts-% aus 2,4-TDA und zu 20 Gewichts-% aus 2,6-TDA besteht, mit 56.75 kg/h flüssigem Phosgen vermischt, das über den äußeren Ringspalt mit einer Geschwindigkeit von 15 m/s zugeführt wird. Die Temperaturen der Eduktströme wurden so eingestellt, dass der aus der Mischdüse austretende Austrittsstrom eine Temperatur von 100°C besitzt. Dieser Strom wurde dann durch einen senkrechtstehendes Rohr mit einem Volumen von 6 1 (Liter) und einem Längen- zu Durchmesserverhältnis von 8 geleitet. Durch eine elektrische Begleitbeheizung wurde die Rohrwand auf eine Temperatur von 140°C eingestellt. Am Kopf des Rohrreaktors wurde die.Flüssigphase von der HCl und Phosgen enthaltenden Gasphase abgetrennt. Die Flüssigphase wurde durch einen weiteren Rohrreaktor mit einem Volumen von 6 1 und einem Längen- zu Durchmesserverhältnis von 8 geleitet. Durch eine elektrische Begleitbeheizung wurde auch hier die Rohrwand auf eine Temperatur von 140°C eingestellt. Am Kopf des Rohrreaktors wurde wieder die Flüssigphase von der HCl und Phosgen enthaltenden Gasphase abgetrennt. Nach der destillativen Entfernung des Phosgens und des Chlorbenzols aus der Flüssigphase wurde TDI bei einer Reinheit von ca. 99,1 % (GC) und einer Ausbeute von 95 % in Bezug auf den eingesetzten TDA-Strom gewonnen. Vor und nach der Synthese musste die Apparatur kontinuierlich mit MCB vorgespült werden. Das Reaktionsvolumen betrug insgesamt ca. 12 1. Der Druck in den Rohrreaktoren wird auf 4.5 bar absolut eingestellt.

Ergebnis: In Vergleichsbeispiel 3 ergibt sich gegenüber dem Beispiel 1 bei gleichem Reaktionsvolumen eine um 2 % geringere Ausbeute des Verfahrens.

**Patentansprüche**

1.   Verfahren zur kontinuierlichen Herstellung von Isocyanaten durch einstufige Umsetzung von primären Aminen mit Phosgen in einer Kaskade aus n Reaktoren, wobei n eine natürliche Zahl von 2 bis 20 darstellt, **dadurch gekennzeichnet, dass** die Vermischung und Umsetzung der Edukte in einem Schritt in einem Temperaturbereich von 60 bis 200°C durchgeführt wird und nach jedem Reaktor die bei der Umsetzung entstehende Gasphase vom Umsetzungsgemisch abgetrennt und die verbleibende Flüssigphase dem nächsten Reaktor zugeführt wird, wobei das Volumen des ersten Reaktors folgender Ungleichung genügt:

$$\text{Volumen }_{\text{Reaktor 1}} < (\text{Volumen }_{\text{Summe der Reaktoren 1 bis n}}) \; / \; \text{n}.$$

2. Verfahren nach Anspruch 1, **dadurch gekennzeichnet, dass** das Volumen des ersten Reaktors das 0,1- bis 0,9-fache von [(Volumen $_{\text{Summe der Reaktoren 1 bis n}}$) / n] beträgt.

3. Verfahren nach Anspruch 1 oder 2, **dadurch gekennzeichnet, dass** es sich bei den Reaktoren um Reaktionsrohre handelt.

4. Verfahren nach Anspruch 3 **dadurch gekennzeichnet, dass** das Umsetzungsgemisch von unten nach oben die senkrecht stehenden Reaktionsrohre durchläuft.

5. Verfahren nach einem der Ansprüche 1 bis 4, **dadurch gekennzeichnet, dass** die Umsetzung in einem Temperaturbereich von 60 bis 200 °C und bei Absolutdrücken von 0,9 bar bis 400 bar durchgeführt wird, wobei das molare Verhältnis von Phosgen zu eingesetzten Aminogruppen 1 : 1 bis 12 : 1 und die Gesamtverweilzeit in den Reaktoren 10 Sekunden bis 3 Stunden beträgt.

6. Verfahren nach einem der Ansprüche 1 bis 5, **dadurch gekennzeichnet, dass** die Abtrennung der Gasphase vom Umsetzungsgemisches am Ende eines Reaktors mittels einer Phasentrennvorrichtung erfolgt, wobei die Phasentrennvorrichtung in den Reaktor integriert ist oder getrennt als Behälter aufgeführt ist.

7. Verfahren nach Anspruch 6, **dadurch gekennzeichnet, dass** zwischen den Phasentrennvorrichtungen und den Reaktoren und/oder am Ende der letzten Phasentrenneinrichtung Druckreduziereinrichtungen verwendet werden.

8. Verfahren nach einem der Ansprüche 1 bis 7, **dadurch gekennzeichnet, dass** der Austrag aus dem letzten Reaktor der Kaskade einer Nachreaktionsvorrichtung zur Nachreaktion zugeführt wird, wobei eine Zersetzung des noch nicht umgesetzten Carbamylchlorids und Aminhydrochlorids erfolgt.

9. Verfahren nach Anspruch 8, **dadurch gekennzeichnet, dass** der Austrag aus dem letzten Reaktor der Kaskade einer Destillationskolonne im Mittelteil zugeführt wird, wobei die Flüssigphase innerhalb der Destillationskolonne von oben nach unten und die Gasphase von unten nach oben geleitet wird.

10. Reaktionsvorrichtung zur kontinuierlichen Herstellung von Isocyanaten durch einstufige Umsetzung von primären Aminen mit Phosgen, aufgebaut aus einer Kaskade aus n Reaktoren, wobei n eine natürliche Zahl von 2 bis 20 darstellt, **dadurch gekennzeichnet, dass** die Reaktoren eine Phasentrennvorrichtung, die zur Abtrennung der bei der Umsetzung entstehende Gasphase geeignet ist, aufweisen, wobei die Phasentrennvorrichtung in den Reaktor integriert ist oder getrennt als Behälter aufgeführt ist, und das Volumen des ersten Reaktors folgender Ungleichung genügt:

$$\text{Volumen }_{\text{Reaktor 1}} < (\text{Volumen }_{\text{Summe der Reaktoren 1 bis n}}) \; / \; \text{n},$$

wobei zwischen Phasentrennvorrichtung und Reaktor eine Druckreduziereinrichtung verwendet wird.

11. Verwendung einer Reaktionsvorrichtung gemäß Anspruch 10 zur Herstellung von aromatischen oder aliphatischen Isocyanaten.

## Claims

1. A process for the continuous preparation of isocyanates by single-stage reaction of primary amines with phosgene in a cascade of n reactors, where n is a natural number from 2 to 20, wherein the gas phase formed in the reaction is separated off from the reaction mixture downstream of each reactor and the remaining liquid phase is passed to the next reactor and the volume of the first reactor conforms to the following inequality:

$$\text{volume}_{\text{reactor 1}} < (\text{volume}_{\text{sum of the reactors 1 to n}}) / n.$$

2. The process according to claim 1, wherein the volume of the first reactor is from 0.1 to 0.9 times [(volume $_{\text{sum of the reactors 1 to n}}$)/n].

3. The process according to claim 1 or 2, wherein the reactors are reaction tubes.

4. The process according to claim 3, wherein the reaction mixture passes through the upright reaction tubes from the bottom upward.

5. The process according to any of claims 1 to 4, wherein the reaction is carried out in a temperature range from 60 to 200°C and at absolute pressures of from 0.9 bar to 400 bar and the molar ratio of phosgene to amino groups used is from 1 : 1 to 12 : 1 and the total residence time in the reactors is from 10 seconds to 3 hours.

6. The process according to any of claims 1 to 5, wherein the gas phase is separated off from the reaction mixture at the end of a reactor by means of a phase separation apparatus which is integrated into the reactor or is present as a separate vessel.

7. The process according to claim 6, wherein pressure reduction devices are used between the phase separation apparatuses and the reactors and/or at the end of the last phase separation apparatus.

8. The process according to any of claims 1 to 7, wherein the output from the last reactor of the cascade is passed to an after-reaction apparatus in which decomposition of the as yet unreacted carbamoyl chloride and amine hydrochloride occurs.

9. The process according to claim 8, wherein the output from the last reactor of the cascade is fed into a distillation column in its middle part, with the liquid phase being passed through the distillation column from the top downward and the gas phase being passed through it from the bottom upward.

10. A reaction apparatus for the continuous preparation of isocyanates by single-stage reaction of primary amines with phosgene, made up of a cascade of n reactors, where n is a natural number from 2 to 20, wherein the reactors are provided with a phase separation apparatus suitable for separating off the gas phase formed in the reaction and the phase separation apparatus is integrated into the reactor or is present as a separate vessel and the volume of the first reactor conforms to the following inequality:

$$\text{volume}_{\text{reactor 1}} < (\text{volume}_{\text{sum of the reactors 1 to n}}) / n.$$

11. The use of a reaction apparatus according to claim 10 for preparing aromatic or aliphatic isocyanates.

**Revendications**

1. Procédé de préparation en continu d'isocyanates par une réaction en une étape d'amines primaires avec du phosgène dans une cascade de n réacteurs, n étant un nombre entier naturel de 2 à 20, **caractérisé en ce que**, après chaque réacteur, la phase gazeuse formée au cours de la réaction est isolée du mélange réactionnel et la phase liquide subsistante est amenée au réacteur suivant, le volume du premier réacteur satisfaisant à l'inéquation suivante :

$$\text{volume}_{\text{réacteur 1}} < (\text{volume}_{\text{somme des réacteurs 1 à n}}) / n.$$

**2.** Procédé suivant la revendication 1, **caractérisé en ce que** le volume du premier réacteur est de 0, 1 à 0,9 fois

$$[\text{volume}_{\text{somme des réacteurs 1 à n}}) \ / \ n].$$

**3.** Procédé suivant la revendication 1 ou 2, **caractérisé en ce que**, pour ce qui concerne les réacteurs, il s'agit de tubes réactionnels.

**4.** Procédé suivant la revendication 3, **caractérisé en ce que** le mélange réactionnel passe du bas vers le haut à travers les tubes réactionnels disposés verticalement.

**5.** Procédé suivant l'une des revendications 1 à 4, **caractérisé en ce que** la réaction est effectuée dans une gamme de températures de 60 à 200°C et à des pressions absolues de 0,9 bar à 400 bars, le rapport molaire entre le phosgène et les groupes amino mis en oeuvre étant de 1/1 à 12/1 et le temps de séjour global dans les réacteurs étant de 10 secondes à 3 heures.

**6.** Procédé suivant l'une des revendications 1 à 5, **caractérisé en ce que** l'isolement de la phase gazeuse à partir du mélange réactionnel a lieu à l'extrémité d'un réacteur au moyen d'un dispositif de séparation de phases, le dispositif de séparation de phases étant intégré dans le réacteur ou introduit séparément sous la forme d'un récipient.

**7.** Procédé suivant la revendication 6, **caractérisé en ce que**, entre les dispositifs de séparation de phases et les réacteurs et/ou à la fin du dernier dispositif de séparation de phases, on utilise des dispositifs de réduction de pression.

**8.** Procédé suivant l'une des revendications 1 à 7, **caractérisé en ce que** l'effluent du dernier réacteur de la cascade est amené à un dispositif postréactionnel pour effectuer une postréaction, une décomposition du chlorhydrate d'amine et du chlorure de carbamyle n'ayant pas encore réagi ayant lieu.

**9.** Procédé suivant la revendication 8, **caractérisé en ce que** l'effluent du dernier réacteur de la cascade est amené à une colonne de distillation, dans la partie centrale, la phase liquide étant, à l'intérieur de la colonne de distillation, conduite du haut vers le bas et la phase gazeuse du bas vers le haut.

**10.** Dispositif réactionnel pour la préparation en continu d'isocyanates par réaction en une étape d'amines primaires avec du phosgène, constitué d'une cascade de n réacteurs, n étant un nombre entier naturel de 2 à 20, **caractérisé en ce que** les réacteurs présentent un dispositif de séparation de phases qui convient pour la séparation de la phase gazeuse formée au cours de la réaction, le dispositif de séparation de phases étant intégré dans le réacteur ou introduit séparément sous la forme d'un récipient, le volume du premier réacteur satisfaisant à l'inéquation suivante :

$$\text{volume}_{\text{réacteur 1}} < (\text{volume}_{\text{somme des réacteurs 1 à n}}) \ / \ n.$$

**11.** Utilisation d'un dispositif réactionnel suivant la revendication 10, pour la préparation d'isocyanates aromatiques ou aliphatiques.